# EUROPEAN PATENT APPLICATION

(11) **EP 4 390 947 A1**
(43) Date of publication of application: **26.06.2024**
(21) Application number: 22216073.1
(22) Date of filing: 22.12.2022
(51) Int. Cl.: G16H 10/60, G16H 15/00, G16H 80/00

(54) **METHODS AND SYSTEMS FOR PREDICTING PATIENT DROPOUT AND ROOT CAUSES FROM REMOTE PATIENT MONITORING**

(71) Applicant: Koninklijke Philips N.V., 5656 AG Eindhoven (NL)
(72) Inventor: LACROIX, Joyca Petra Wilma, Eindhoven (NL); VAN BERKEL, Joep, Eindhoven (NL); VELTHOVEN (ep. DESSAUD), Nathalie, 5656AG Eindhoven (NL); DONGRE, Chaitanya, Eindhoven (NL)
(74) Representative: Philips Intellectual Property & Standards

(57) **Abstract**

The present disclosure is directed to methods and systems for predicting patient dropout from a remote patient monitoring (RPM) program, as well as root dropout causes, based on clinical features using a "dropout prediction engine". As described herein, the methods and systems address the clinical challenge of early detection of dropout risk of patients from these virtual care programs through a data-driven approach that accurately identifies the likely root cause(s) of the dropout and enables the prevention of the dropout by applying timely interventions targeting the root causes of the dropout. As a result, dropout prevention effectuated through targeted interventions will promote continued engagement with virtual care, thereby leading to lower costs of care, better health outcomes, and better patient and staff experience.

## Description

### FIELD OF THE DISCLOSURE

The present disclosure relates generally to methods and systems for predicting dropout from a remote patient monitoring program, and more specifically to methods and systems for predicting dropout and root causes for the dropout.

### BACKGROUND

Widespread adoption of electronic health records has enabled automated data capturing and propelled predictive risk modeling in a variety of respects and across many different cohorts. In certain settings, risk modeling has become an essential pillar for measuring outcomes and other benchmarking. These and other technological advancements (e.g., advances in monitoring hubs, smartphone platforms, sensor technologies, cellular networks, cloud computing, and mobile and wearable medical devices) have removed many of the technical barriers to successful remote patient monitoring (RPM) and remote patient healthcare in general. Further, the rising geriatric population and the growing need to expand healthcare access, cost benefits of telehealth and RPM, benefits of RPM to reduce the burden on medical resources, advancements in telecommunications, growing incidences of chronic diseases, and increasing investments in telehealth and RPM are the major factors driving the growth of this market. As a result of these technological innovations and market factors, adoption and scaling of RPM has greatly accelerated.

In particular, RPM programs provide care teams with the tools they need to remotely track the health of their patients at home, collaborate with the patients' doctors and help detect problems before they lead to readmissions. Providers can assign patients to specific care protocols and interventions that are tailored to condition or acuity level, which can include measuring vital signs, completing surveys, watching educational videos and participating in video visits with the virtual care team. However, challenges towards successful deployment of RPM solutions remain and a key remaining challenge is that a substantial portion of patients will drop out early of the program.

### SUMMARY OF THE DISCLOSURE

According to an embodiment of the present disclosure, a method for predicting a likelihood of dropout and a root dropout cause for a patient under remote monitoring using a dropout prediction system is provided. The method comprises: obtaining, from an electronic patient records database, a plurality of records for a patient under remote monitoring by a care provider; generating a chronological snapshot of the patient, wherein the chronological snapshot includes a plurality of dropout prediction features extracted from the plurality of records obtained from the electronic patient records database; generating a likelihood of dropout for the patient by comparing the chronological snapshot of the patient with at least one of a clinically-validated care pathway, an earlier chronological snapshot of the patient; and one or more similar patient snapshots; predicting a root dropout cause for the patient based on one or more of the dropout prediction features of the chronological snapshot of the patient; determining one or more recommended interventions tailored to the patient based on the generated likelihood of dropout and the predicted root dropout cause, wherein the one or more recommended interventions are intended to prevent dropout of the patient from remote monitoring by the care provider; and presenting to the care provider, via a care provider interface of the dropout prediction system, the one or more recommended interventions.

In an aspect, generating a likelihood of dropout for the patient includes generating a first likelihood of dropout for the patient corresponding to a first future time and generating a second likelihood of dropout for the patient corresponding to a second future time; wherein predicting a root dropout cause for the patient includes predicting a first root dropout cause for the patient corresponding to the first future time and predicting a second root dropout cause for the patient corresponding to the second future time; and wherein the one or more recommended interventions include a first recommended intervention determined based on the first likelihood of dropout generated for the patient and the first root dropout cause predicted for the patient, and a second recommended intervention determined based on the second likelihood of dropout generated for the patient and the second root dropout cause predicted for the patient.

In an aspect, the first future time is between 1 and 14 days from a current time, and the second future time is between 1 and 6 months from the current time.

In an aspect, the plurality of records for the patient under remote monitoring by the care provider include at least one of: identification information for the patient; medical history for the patient; treatment history for the patient; medical directives for the patient; unique identifiers/fingerprints of patient's virtual trajectory through the patient interface based on abstraction of virtual movements represented in a virtual trajectory of human-machine interaction within patient interfacing computer program or application; and one or more physiological measurements taken from the patient.

In an aspect, the plurality of records for the patient under remote monitoring by the care provider further includes at least one of: virtual care solution usage information; a technology affinity measured for the patient; and feedback information from one or more historical or concurrent patients.

In an aspect, generating a likelihood of dropout for the patient by comparing the chronological snapshot of the patient with one or more similar patient snapshots includes comparing the chronological snapshot of the patient with one or more similar patient snapshots using an unsupervised hierarchical clustering technique.

In an aspect, the plurality of dropout prediction features extracted from the plurality of records includes at least one of: a response time from the care provider to a patient-initiated action; a history of challenges involving use of a virtual care solution by the patient; clinical trends for the patient; user interaction data for the patient; and portal use data showing frequency of use of specific components over time.

In an aspect, the root dropout cause for the patient is predicted based on one or more of the dropout prediction features of the chronological snapshot of the patient using an intervention component.

In an aspect, the method further comprises: recording, in the electronic patient records database, the implementation of one or more of the recommended interventions by the care provider; determining an impact of the one or more recommended interventions implemented by the care provider on the plurality of dropout prediction features extracted from the plurality of records; and updating the intervention component based on the impact determined for the one or more recommended interventions implemented by the care provider.

In an aspect, the method further comprises: determining a level of accuracy for the root dropout cause predicted for the patient; and if the level of accuracy is below a threshold: automatically initiating a patient outreach action via a patient interface of the dropout prediction system; receiving, via the patient interface, a response from the patient, wherein the response indicates a true dropout cause for the patient; and recording, in the electronic patient records database, the response; and updating a dropout prediction engine based on the response received via the patient interface.

According to another embodiment of the present disclosure, a dropout prediction system configured to generate a likelihood of dropout and predict a root dropout cause for a patient undergoing remote patient monitoring by a care provider is provided. The system comprises: an electronic patient records database comprising a plurality of records for a plurality of patients under remote monitoring by the care provider; a dropout prediction engine configured to generate a likelihood of dropout for the patient and to predict a root dropout cause for the patient; one or more processors configured to: (i) obtain, from the electronic patient records database, a plurality of records for the patient under remote monitoring by the care provider; (ii) generate a chronological snapshot of the patient, wherein the chronological snapshot includes a plurality of dropout prediction features extracted from the plurality of records obtained from the electronic patient records database; (iii) generate the likelihood of dropout for the patient by comparing the chronological snapshot of the patient with at least one of: a clinically-validated care pathway, an earlier chronological snapshot of the patient; and one or more similar patient snapshots; (iv) predict a root dropout cause for the patient based on one or more of the dropout prediction features of the chronological snapshot of the patient; (v) determine one or more recommended interventions tailored to the patient based on the generated likelihood of dropout and the predicted root dropout cause, wherein the one or more recommended interventions are intended to prevent dropout of the patient from remote monitoring by the care provider; and (vi) present to the care provider, via a care provider interface, the one or more recommended interventions; and a care provider interface configured to present the likelihood of dropout generated for the patient, the root dropout cause predicted for the patient, and the one or more recommended interventions determined for the patient.

In an aspect, generating a likelihood of dropout for the patient includes generating a first likelihood of dropout for the patient corresponding to a first future time and generating a second likelihood of dropout for the patient corresponding to a second future time; wherein predicting a root dropout cause for the patient includes predicting a first root dropout cause for the patient corresponding to the first future time and predicting a second root dropout cause for the patient corresponding to the second future time; and wherein the one or more recommended interventions include a first recommended intervention determined based on the first likelihood of dropout generated for the patient and the first root dropout cause predicted for the patient, and a second recommended intervention determined based on the second likelihood of dropout generated for the patient and the second root dropout cause predicted for the patient.

In an aspect, the dropout prediction engine is configured to generate a likelihood of dropout for the patient by comparing the chronological snapshot of the patient with one or more similar patient snapshots using an unsupervised hierarchical clustering technique.

In an aspect, the dropout prediction engine is configured to predict the root dropout cause for the patient based on one or more of the dropout prediction features of the chronological snapshot of the patient using a trained dropout prediction model, the dropout prediction model being trained on a training dataset by a machine learning algorithm.

In an aspect, the one or more processors are further configured to: (vii) record, in the electronic patient records database, the implementation of one or more of the recommended interventions by the care provider; (viii) determine an impact of the one or more recommended interventions implemented by the care provider on the plurality of dropout prediction features extracted from the plurality of records; and (ix) update an intervention component based on the impact determined for the one or more recommended interventions implemented by the care provider.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a flowchart illustrating a method for predicting a likelihood of dropout and a root dropout cause for a patient under remote monitoring according to aspects of the present disclosure.
FIG. 2 is a flowchart illustrating additional features of the method for predicting a likelihood of dropout and a root dropout cause for a patient under remote monitoring according to aspects of the present disclosure.
FIG. 3 is another flowchart illustrating additional features of the method for predicting a likelihood of dropout and a root dropout cause for a patient under remote monitoring according to aspects of the present disclosure.
FIG. 4 is a diagram illustrating the deployment of a dropout prediction system according to aspects of the present disclosure.
FIG. 5 is a block diagram illustrating a portion of the dropout prediction system according to aspects of the present disclosure.

### DETAILED DESCRIPTION OF EMBODIMENTS

The present disclosure is directed to methods and systems for predicting patient dropout from a remote patient monitoring (RPM) program, as well as root dropout causes, based on clinical features using a dropout prediction engine. As described herein, the methods and systems address the clinical challenge of early detection of dropout risk of patients from these virtual care programs through a data-driven approach that accurately identifies the likely root cause(s) of the dropout and enables the prevention of the dropout by applying timely interventions targeting the root causes of the dropout. As a result, dropout prevention effectuated through targeted interventions will promote continued engagement with virtual care, thereby leading to lower costs of care, better health outcomes, and better patient and staff experience.

The embodiments and implementations disclosed or otherwise envisioned herein can be utilized with any virtual patient care system, including but not limited to clinical decision support tools, among other systems. For example, one application of the embodiments and implementations herein is to improve systems such as, e.g., the Philips^{®} BioTel, Philips^{®} Engage, SRC digital and remote care solutions, and the like, among many others. However, the disclosure is not limited to these devices or systems, and thus disclosure and embodiments disclosed herein can encompass any device or system capable of remote patient monitoring.

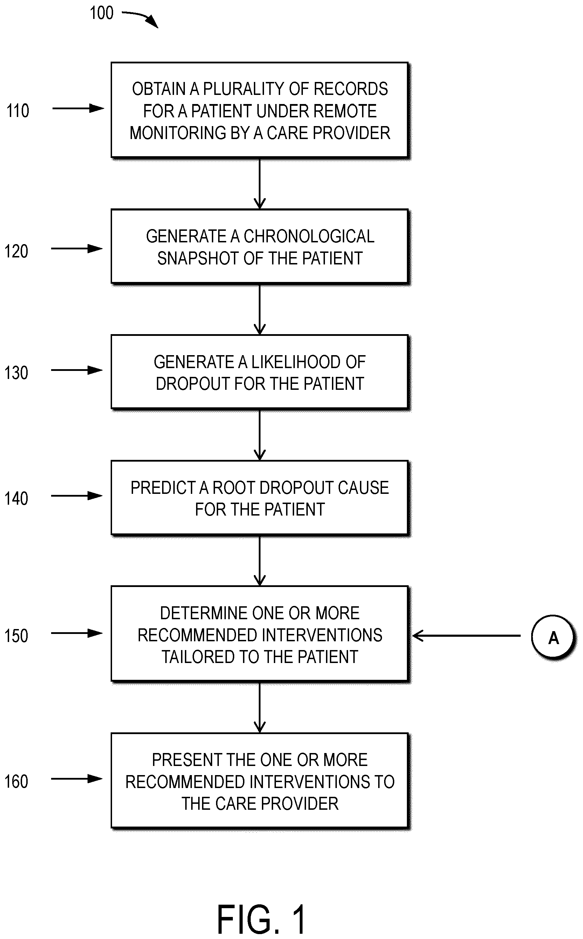

Turning to FIG. 1, a flowchart of a method 100 for predicting a likelihood of dropout and a root dropout cause for a patient under remote monitoring using a dropout prediction system is illustrated according to aspects of the present disclosure. The dropout prediction system can be any of the systems described or otherwise envisioned herein. For example, as discussed in greater detail below, the dropout prediction system 410 can be configured predict a likelihood of dropout and a root dropout cause for a patient under remote monitoring. In embodiments, the dropout prediction system 410 can include a "dropout prediction engine" 264 configured to analyze a plurality of different predefined dropout prediction features to generate a likelihood of dropout and/or a root dropout cause for the patient.

At a step 110, the method 100 includes obtaining a plurality of records for a patient in an RPM program. In some embodiments, the plurality of records for the patient may be obtained by the dropout prediction system 410. In further embodiments, the plurality of records for the patient may be obtained from an electronic database 402 where all the available patient data relevant for dropout prediction and root cause prediction are stored and/or accessible. For example, the electronic database 402 may include physiologic, diagnosis, and treatment information (collectively, "medical information" or "medical data") for a plurality of patients under one or more RPM programs administered by one or more care providers. That is, the electronic database 402 can comprise a plurality of healthcare-related records for a plurality of patients, including historical patients and/or patients of one or more current RPM programs.

In some embodiments, the electronic database 402 can include self-reported data on medical conditions (existing or new), physical health, and mental health for the patient. Put another way, the electronic database 402 can include medical data generated and reported by the patient, including but not limited to, updated physiological measurements and test results.

In further embodiments, the electronic database 402 can also include patterns of use of the virtual care system used in the patient's RPM program, including but not limited to, virtual care platforms, virtual health assistants, electronic and/or outpatient medical devices, virtual visit systems, medical appointment scheduling systems, and the like. In embodiments, the electronic database 402 includes patterns of medical data (e.g., test results, hospital visits, provider interactions), as well as other engagement-related features such as medication adherence.

In embodiments, the electronic database 402 may also include subjective data that is collected, self-reported, and/or assessed over time, including but not limited to, the patient's comfortability and/or affinity with technology.

At a step 120, the method 100 includes generating a chronological snapshot of the patient. In embodiments, the chronological snapshot of the patient represents the patient's health journey based on the data accessible via the database 402 and obtained in step 110 of the method 100. In some embodiments, the chronological snapshot includes a plurality of dropout prediction features extracted from the plurality of records obtained from the electronic database 402. For example, in some embodiments, the plurality of dropout prediction features may include one or more of a response time from the care provider to a patient-initiated action, a history of challenges involving use of a virtual care solution by the patient, clinical trends for the patient, user interaction data for the patient, and/or portal use data showing frequency of use of specific components over time.

In certain embodiments, the chronological snapshot of the patient may encompass a predetermined time period. For example, in some embodiments, the chronological snapshot may include one or more dropout prediction features taken across the entire time window of data obtained for the patient. In other embodiments, the chronological snapshot may include a plurality of dropout prediction features specific to a particular care episode. That is, the chronological snapshot may include dropout prediction features within a subset of the total time window for which data from the database 402 is available for the patient.

At a step 130, the method 100 includes generating a likelihood of dropout for the patient based on the patient's chronological snapshot. For example, in embodiments, the likelihood of dropout for the patient may be generated by comparing the chronological snapshot of the patient with at least one of a clinically-validated care pathway, an earlier chronological snapshot of the patient, and/or one or more similar patient snapshots. In particular embodiments, the likelihood of dropout for the patient may be generated by a dropout prediction engine (e.g., dropout prediction engine 563), which may comprise a trained dropout prediction machine learning model. That is, the trained dropout prediction model may be trained by a machine learning algorithm on a training dataset that comprises a plurality of medical records for a plurality of historical patients.

In particular embodiments, similar patients and/or similar patient cohorts may be identified through clustering of historical patients with records stored in or accessible to the database 402. In some embodiments, the identification of similar patients and/or similar patient cohorts may be performed using an unsupervised hierarchical clustering algorithm, where subsequent and/or new patients are assigned to a cluster based on some distance measure along the feature space. In embodiments, the feature space may comprise one or more of the dropout prediction features.

In some embodiments, the likelihood of dropout predicted for a patient may be compared with a dropout threshold. In embodiments, if the likelihood of dropout exceeds the dropout threshold, then the rest of the method 100 may be triggered. Put another way, if the likelihood of dropout is below a predefined dropout threshold, then the method 100 may end or may pause until the likelihood of dropout is determined to exceed the dropout threshold. In embodiments, the dropout threshold may be predefined based on the particular remote patient monitoring program, the care provider protocols, the severity of the patient's condition, a statutory requirement set by a governmental agency, a reimbursement schedule set by an insurance carrier, and/or the like.

In embodiments, generating a likelihood of dropout for the patient can include generating a likelihood of dropping out at several moments in the future (i.e., a certain future time measured from the time of the prediction). For example, when generating a likelihood of dropout for the patient, the likelihood may correspond to a particular time frame, such as a likelihood of dropout within the next 1 to 14 days. Put another way, at the step 130, the method 100 can include generating a first likelihood of dropout for the patient corresponding to a first future time, and generating at least a second likelihood of dropout for the patient corresponding to at least a second future time. In embodiments, the future time of interest may be, for example and without limitation, 1 day, 7 days, 14 days, 1 month, 2 months, and/or 3 months. In some embodiments, the first future time is between 1 and 14 days from a current time (i.e., a time of execution of step 130), while the second future time is between 1 and 6 months from the current time (i.e., the time of execution of step 130).

In a step 140, the method 100 includes predicting a root dropout cause for the patient based on one or more of the dropout prediction features of the chronological snapshot of the patient. In some embodiments, the root dropout cause for the patient may be predicted by identifying the occurrence of a set of specific root causes known to influence dropout. These specific root causes can include, but are not limited to, long response times for the care provider to patient activity (e.g., response to an uploaded measurement) or requests for interaction (e.g., submission of a question via virtual care messaging platform), frequency and trend of occurrence of specific problems (e.g., login problems, problems uploading a measurement, problems using the chat features, problems accessing earlier measurements), usability problems manifesting as above-average times or effort needed to get certain tasks accomplished (e.g., time or number of clicks the patient uses to upload a measurement), clinical trends showing the patient is in a stable or unstable phase, subjective data and/or interaction data (e.g., assessing the experience of the patient with the virtual care system), and/or virtual care system use data showing the frequency of use of specific components over time (e.g., the frequency of downloading healthcare content or articles, the frequency and type of uploaded measurements).

In embodiments, a repository of root causes may be maintained in one or more databases, such as an independent database, and can include a listing of potential dropout causes. For example, the repository of root causes can include, but is not limited to, long response times for the care provider to patient activity (e.g., response to an uploaded measurement) or requests for interaction (e.g., submission of a question via virtual care messaging platform), frequency and trend of occurrence of specific problems (e.g., login problems, problems uploading a measurement, problems using the chat features, problems accessing earlier measurements), usability problems manifesting as above-average times or effort needed to get certain tasks accomplished (e.g., time or number of clicks the patient uses to upload a measurement), clinical trends showing the patient is in a stable or unstable phase, subjective data and/or interaction data (e.g., assessing the experience of the patient with the virtual care system), and/or virtual care system use data showing the frequency of use of specific components over time (e.g., the frequency of downloading healthcare content or articles, the frequency and type of uploaded measurements).

In embodiments, the repository of root causes can be allocated to a specific patient in a plurality of combinations and/or chronological order and weights in terms of likelihood. The accuracy of the root cause detection can be continuously improved as the volume of potential root causes is increased with time, by a targeted stimulus-response mechanism (e.g., a patient feedback mechanism), as well as additional data collection.

In embodiments, predicting a root dropout cause for the patient can include predicting a root dropout cause for the patient at several moments in the future (i.e., a certain future time measured from the time of the prediction). For example, when predicting a root dropout cause for the patient, the predicted root cause may correspond to a particular time frame, such as a predicted root cause of dropout within the next 1 to 14 days. Put another way, at the step 140, the method 100 can include predicting a root dropout cause for the patient corresponding to a first future time, and generating at least a second predicting a root dropout cause for the patient corresponding to at least a second future time. In embodiments, the future time of interest may be, for example and without limitation, 1 day, 7 days, 14 days, 1 month, 2 months, and/or 3 months. In some embodiments, the first future time is between 1 and 14 days from a current time (i.e., a time of execution of step 130), while the second future time is between 1 and 6 months from the current time (i.e., the time of execution of step 130).

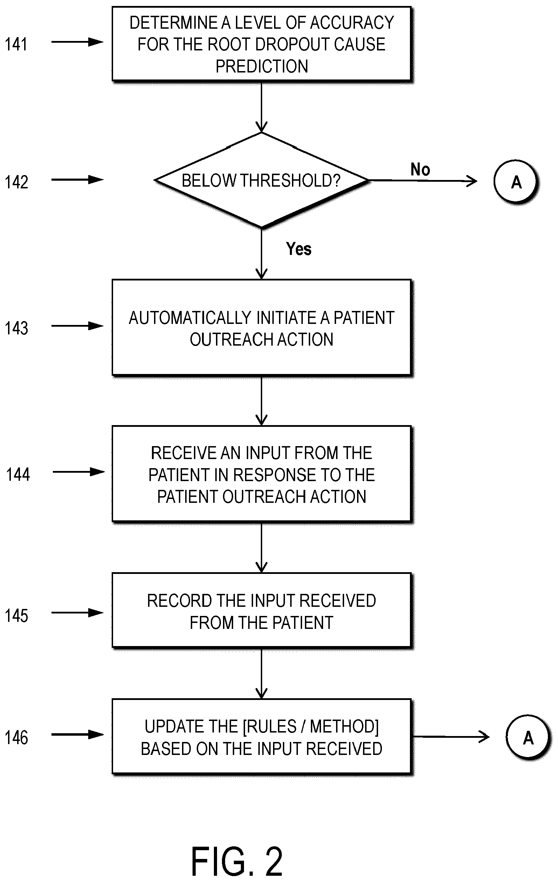

With reference to FIG. 2, the method 100 may also include one or more steps to validate an accuracy level of at least one predicted root dropout cause. For example, in a step 141, the method 100 includes determining a level of accuracy or confidence for the root dropout cause predicted in step 140. In a step 142, the method 100 includes determining whether the level of accuracy or confidence is below a predetermined threshold. If so, the method 100 may proceed to step 150. Otherwise, the method 100 may include, in a step 143, automatically initiating a patient outreach action via a patient interface of the dropout prediction system 410. In a step 144, the method 100 includes receiving input from the patient via the patient interface, in response to the patient outreach action, wherein the input / response indicates a true dropout cause for the patient. In a step 145, the method 100 includes recording the input / response in the electronic database 402. In a step 146, the method 100 includes updating the dropout prediction engine and/or the repository of dropout causes based on the received response.

In a step 150, the method 100 includes determining one or more recommended interventions tailored to the patient based on the generated likelihood of dropout and the predicted root dropout cause. In embodiments, the one or more recommended interventions are intended to prevent dropout of the patient from the RPM program provided by the care provider.

In embodiments, the one or more interventions may be selected by the care provider from a library of possible, proven, validated interventions in order to bring the dropout risk down to below a dropout threshold relevant for the managed patient population.

In embodiments, determining one or more recommended interventions can include determining one or more recommended interventions to take or perform at over a period of time in the future (i.e., a certain future time measured from the time of the prediction). For example, the one or more recommended interventions may include an immediate intervention, a short-term intervention, and/or a long-term intervention. In further embodiments, determining one or more recommended interventions can include determining one or more recommended interventions for each predicted root dropout cause and/or likelihood of dropout for the patient.

In a step 160, the method 100 includes presenting the one or more recommended interventions determined in step 150 to the care provider. In embodiments, the one or more recommended interventions may be presented via a care provider interface of the dropout prediction system 410.

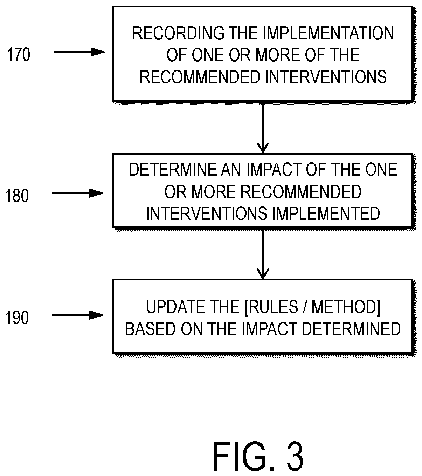

With reference to FIG. 3, the method 100 can further include: in a step 170, recording the implementation of one or more of the recommended interventions; in a step 180, determining an impact of one of more of the recommended interventions implemented; and, in a step 190, updating an intervention component (e.g., intervention component 564) based on the impact determined for the one or more recommended interventions.

For example, in embodiments, step 170 of the method 100 includes creating a record in the electronic database 402 that stores information about any interventions taken to prevent a patient dropout from occurring. In specific embodiments, step 170 includes recording information in the electronic database 402 regarding which interventions were recommended and which interventions were implemented by, for example, the care provider and/or the virtual care system.

In embodiments, step 180 of the method 100 includes determining an impact of the one or more interventions that are implemented. For example, in some embodiments, an impact of implementing one or more recommended interventions may be determined for each of the one or more dropout prediction features used to determine the likelihood of dropout for the patient.

In embodiments, step 190 of the method 100 includes updating intervention component (e.g., intervention component 564) based on the impact determined for the one or more recommended interventions.

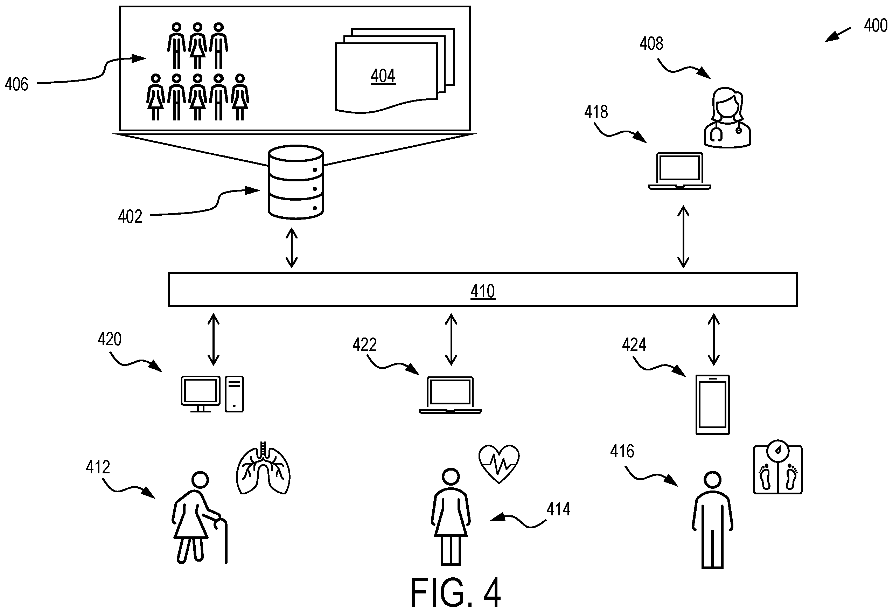

With reference to FIG. 4, a remote patient monitoring environment 400 is illustrated wherein a virtual care platform having a dropout prediction system 410 is deployed in accordance with aspects of the present disclosure. The dropout prediction system 410 can be configured to generate a likelihood of dropout for a patient and/or predict a root dropout cause for the patient, as described herein. In some embodiments, the dropout prediction system 410 may be at least part of a large patient data management system (PDMS) and/or a virtual patient management solution or RPM system.

In the example of FIG. 4, three patients 412, 414, 416 are undergoing a remote patient monitoring protocol being administered by a care provider 408. These patients 412, 414, 416 may be under the same protocol or different protocols, for example, for differing conditions (e.g., respiratory issues in the case of a first patient 412, cardiac issues in the case of a second patient 414, and/or weight issues in the case of a third patient 416). In embodiments, the dropout prediction system 410 may be used to monitoring such patients 412, 414, 416 for potential dropout and dropout causes.

As shown, the dropout prediction system 410 comprises an electronic patient records database 402 storing a plurality of records 404 for a plurality of patients 406. In embodiments, the plurality of patients 406 can include patients currently active in an RPM program administered by a care provider 408, but can also include historical patients (i.e., patients no longer active in an RPM program).

In embodiments, the plurality of records may include identification information for one or more patients, medical histories for one or more patients, treatment histories for one or more patients, medical directives for one or more patients, and/or physiological measurements or lab results for one or more patients. In some embodiments, the plurality of records 404 may include self-reported data on medical conditions (existing or new), physical health, and mental health for one or more patients. Put another way, the plurality of records 404 can include medical data generated and reported by one or more patients, including but not limited to, updated physiological measurements and test results.

In further embodiments, the plurality of records 404 can also include patterns of use of the virtual care system used in the patient's RPM program, including but not limited to, virtual care platforms, virtual health assistants, electronic and/or outpatient medical devices, virtual visit systems, medical appointment scheduling systems, and the like. In embodiments, the plurality of records 404 include patterns of medical data (e.g., test results, hospital visits, provider interactions), as well as other engagement-related features such as medication adherence. The plurality of records 404 may also include subjective data that is collected, self-reported, and/or assessed over time, including but not limited to, the patient's comfortability and/or affinity with technology, feedback from one or more historical or concurrent patients, and the like.

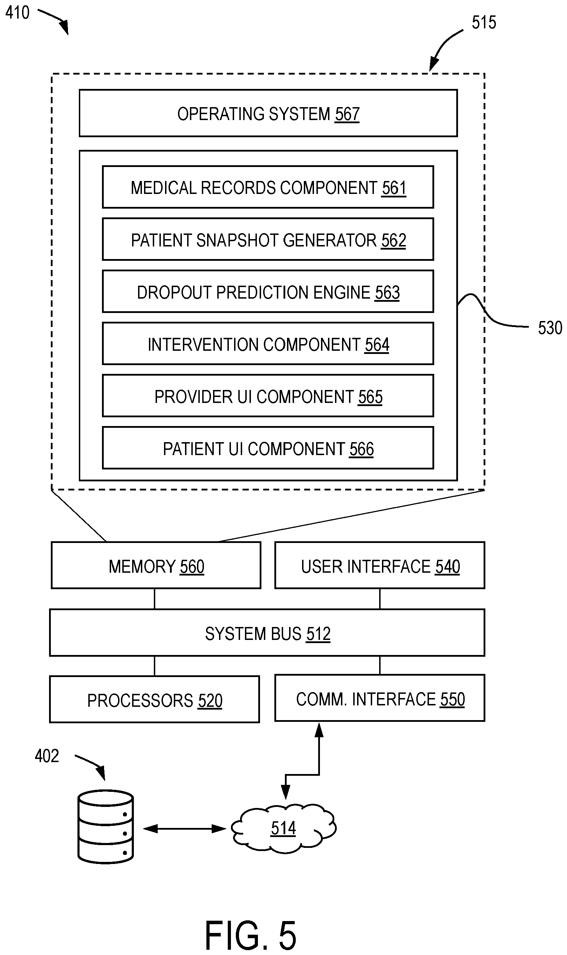

With reference to FIG. 4 and FIG. 5, the dropout prediction system 410 can include a dropout prediction engine 562 and one or more processors 520 configured to perform one or more steps of the methods described herein. In embodiments, the dropout prediction engine 562 and the one or more processors 520 may form part of a larger virtual care platform that is in communication with the electronic database 402. In particular embodiments, the dropout prediction system 410 may comprise one or more processors 520, machine-readable memory 560, a user interface 540, and/or a communications interface 550, all of which may be interconnected and/or communication through a system bus 512 containing conductive circuit pathways through which instructions (e.g., machine-readable signals) may travel to effectuate communication, tasks, storage, and the like.

As discussed herein, the one or more processors 520 may be configured to perform one or more steps of the methods described herein, including but not limited to, the following: a processor configured to: (i) obtain, from the electronic patient records database 402, a plurality of records 404 for a patient (e.g., patients 412, 414, 416) under remote monitoring by the care provider 408; (ii) generate a chronological snapshot of the patient (e.g., patients 412, 414, 416), wherein the chronological snapshot includes a plurality of dropout prediction features extracted from the plurality of records 404 obtained from the electronic patient records database 402; (iii) generate the likelihood of dropout for the patient by comparing the chronological snapshot of the patient (e.g., patients 412, 414, 416) with at least one of: a clinically-validated care pathway, an earlier chronological snapshot of the patient; and one or more similar patient snapshots; (iv) predict a root dropout cause for the patient (e.g., patients 412, 414, 416) based on one or more of the dropout prediction features of the chronological snapshot of the patient; (v) determine one or more recommended interventions tailored to the patient based on the generated likelihood of dropout and the predicted root dropout cause, wherein the one or more recommended interventions are intended to prevent dropout of the patient from remote monitoring by the care provider; and (vi) present to the care provider 408, via a care provider interface 418, the one or more recommended interventions.

In some examples, the one or more processors 520 may include a high-speed data processor adequate to execute the program components described herein and/or various specialized processing units as may be known in the art. In some examples, the one or more processors 520 may be a single processor, multiple processors, or multiple processor cores on a single die.

In some examples, the communications interface 550 can include a network interface configured to connect the dropout prediction system 410 to a communications network 514, an input/output ("I/O") interface configured to connect and communicate with one or more peripheral devices, a memory interface configured to accept, communication, and/or connect to a number of machine-readable memory devices, and the like.

In certain embodiments, the communications interface 550 may operatively connect the coordinating system 410 to a communications network 514, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some examples, coordinating system 410 may communicate with one or more remote / cloud-based servers (e.g., the electronic medical records database 402), cloud-based services, and/or remote devices via the communications network 514.

The memory 560 can be variously embodied in one or more forms of machine-accessible and machine-readable memory. In some examples, the memory 560 includes a storage device that comprises one or more types of memory. For example, a storage device can include, but is not limited to, a non-transitory storage medium, a magnetic disk storage, an optical disk storage, an array of storage devices, a solid-state memory device, and the like, including combinations thereof.

Generally, the memory 560 is configured to store data / information and instructions 215 that, when executed by the one or more processors 220, causes the dropout prediction system 410 to perform one or more tasks. In particular examples, the memory 560 includes a dropout prediction package 530 that causes the dropout prediction system 410 to perform one or more steps of the methods described herein.

In embodiments, the dropout prediction package 530 comprises a collection of program components, database components, and/or data. Depending on the particular implementation, the dropout prediction package 530 may include software components, hardware components, and/or some combination of both hardware and software components.

The dropout prediction package 530 may include one or more software packages configured to predict a likelihood of dropout for a patient and/or root dropout causes. These software packages may be incorporated into, loaded from, loaded onto, or otherwise operatively available to and from the dropout prediction system 410.

In some examples, the dropout prediction package 530 and/or one or more individual software packages may be stored in a local storage device 560. In other examples, the dropout prediction package 530 and/or one or more individual software packages may be loaded onto and/or updated from a remote server via the communications interface 550.

In particular embodiments, the dropout prediction package 530 can include, but is not limited to, instructions having a medical records component 561, a patient snapshot generator 562, a dropout prediction engine 563, an intervention component 564, a care provider user interface (UI) component 565, and/or a patient UI component 566. These components may be incorporated into, loaded from, loaded onto, or otherwise operatively available to and from the dropout prediction system 410.

In embodiments, the medical records component 560 can be a stored program component that is executed by at least one processor, such as the one or more processors 520 of the dropout prediction system 410. In particular, the medical records component 560 can be configured to interface with an electronic medical records database 402 in order to obtain a plurality of records 404 for one or more patients 406, as described herein. That is, the medical records component 560 may be configured to request, receive, and/or otherwise obtain a plurality of medical records for one or more patients 406.

In embodiments, one or more of the patients 406 may be historical patients. In other embodiments, one or more of the patients may be current RPM program patients 412, 414, 416. In still further embodiments, the medical records component 560 may obtain a plurality of records for a combination of historical and/or current RPM program patients 412, 414, 416.

In embodiments, the patient snapshot generator 562 can be a stored program component that is executed by at least one processor, such as the one or more processors 520 of the dropout prediction system 410. In particular, the dropout prediction system 410 can be configured to extract a plurality of different predefined dropout prediction features for a patient and generate a chronological snapshot of the patient, as described herein. In particular, the patient snapshot generator 562 can be configured to extract predefined dropout prediction features from the plurality of records 404 obtained from an electronic medical records database 402 using natural language processing and/or a machine learning algorithm.

As described above, the chronological snapshot of the patient represents the patient's health journey based on the data accessible via the database 402. For example, in some embodiments, the plurality of dropout prediction features may include one or more of a response time from the care provider to a patient-initiated action, a history of challenges involving use of a virtual care solution by the patient, clinical trends for the patient, user interaction data for the patient, and/or portal use data showing frequency of use of specific components over time.

In certain embodiments, the chronological snapshot of the patient may encompass a predetermined time period. For example, in some embodiments, the chronological snapshot may include one or more dropout prediction features taken across the entire time window of data obtained for the patient. In other embodiments, the chronological snapshot may include a plurality of dropout prediction features specific to a particular care episode. That is, the chronological snapshot may include dropout prediction features within a subset of the total time window for which data from the database 402 is available for the patient.

In embodiments, the dropout prediction engine 563 can be a stored program component that is executed by at least one processor, such as the one or more processors 520 of the dropout prediction system 410. In particular, the dropout prediction engine 563 can be configured to analyze the chronological snapshot of one or more patients, including but not limited to the extracted plurality of different dropout prediction features, and generate a likelihood of dropout and/or a predicted root dropout cause, as described herein.

In some embodiments, the dropout prediction engine 563 may also be configured to validate an accuracy level of at least one predicted root dropout cause. For example, the dropout prediction engine 563 may determine a level of accuracy or confidence for the root dropout cause predicted, determine whether the level of accuracy or confidence is below a predetermined threshold, and if not, automatically initiate a patient outreach action via a patient interface 420, 422, 424 of the dropout prediction system 410. As described herein, the patient UI component 566 may be configured to receive input from the patient 412, 414, 416 via the patient interface 420, 422, 424, in response to the patient outreach action, wherein the input / response indicates a true dropout cause for the patient. Then, the dropout prediction engine 563 may record the input / response in the electronic database 402 and/or update the dropout prediction engine 563 based on the received response.

In embodiments, the intervention component 564 can be a stored program component that is executed by at least one processor, such as the one or more processors 520 of the dropout prediction system 410. In particular, the intervention component 564 can be configured to analyze (i) the chronological snapshot of one or more patients, including but not limited to the extracted plurality of different dropout prediction features, (ii) the generated likelihood of dropout for the one or more patients, and (iii) the predicted root dropout cause for the one or more patients, and determining based thereon one or more recommended interventions tailored to the one or more patients, wherein the one or more recommended interventions are intended to prevent dropout of the one or more patients from a RPM program administered by a care provider.

In some embodiments, the intervention component 564 can also be configured to create records in the electronic database 402 that stores information about any interventions taken to prevent a patient dropout from occurring. In further embodiments, the intervention component 564 may be configured to determine an impact of the one or more interventions that are implemented. For example, in some embodiments, an impact of implementing one or more recommended interventions may be determined for each of the one or more dropout prediction features used to determine the likelihood of dropout for the patient. Then, the intervention component 564 may be updated based on the impact determined for the one or more recommended interventions.

In embodiments, the dropout prediction system 410 can include a care provider user interface 418 and/or a patient user interface 420, 422, 424, and the dropout prediction package 530 further includes a care provider user interface (UI) component 565 and/or a patient UI component 566 configured to operate these user interfaces 418, 420, 422, 424.

For example, the care provider UI component 565 can be a stored program component that is executed by at least one processor, such as the one or more processors 520 of the dropout prediction system 410. In particular, the care provider UI component 565 can be configured operate a care provider user interface 418 in order to present information to a care provider 408, as described herein. In some embodiments, the care provider UI component 565 can include a programmable processor, such as a graphics processing units (GPU), which is specialized for rendering images on a monitor or display screen of a user interface 418. In other words, the user interface 418 may be configured, via a care provider UI component 565, to provide or otherwise present a likelihood of dropout and/or root dropout cause generated for one or more patients.

In embodiments, the patient UI component 566 can be a stored program component that is executed by at least one processor, such as the one or more processors 520 of the dropout prediction system 410. In particular, the patient UI component 566 can be configured operate a patient user interface 420, 422, 424 in order to present information to a patient 412, 414, 416 408, as described herein. In some embodiments, the patient UI component 566 can include a programmable processor, such as a graphics processing units (GPU), which is specialized for rendering images on a monitor or display screen of a user interface 420, 422, 424. In particular embodiments, the user interface 420, 422, 424 may be configured, via a care provider UI component 566, to provide or otherwise present one or more outreach / intervention attempts to the patient 412, 414, 416.

In particular embodiments, the patient UI component 566 may be configured to request specific additional information from the patients 412, 414, 416 to be able to gain certainty related to the root cause prediction by asking a couple of questions (e.g., "we observe that you upload less and less measurements, can you please help us understand the reason?"), and making specific recommendations to the patient 412, 414, 416 to decrease the risk of dropout (e.g., if the predicted likely root cause is that the patient failed to login a couple of times, the system may provide tips and tricks for easy login).

The dropout prediction system 410 may also include an operating system component 567, which may be stored in the memory 560. The operating system component 567 may be an executable program facilitating the operation of the dropout prediction system 410. Typically, the operating system component 567 can facilitate access of the communications interface 550, and can communicate with other components of the dropout prediction system 410, including but not limited to, the user interface 540 (including but not limited to the provider UIs 418 and/or the patient UIs 420, 422, 424), the memory 560, and/or the electronic medical records database 402.

It should be appreciated that all combinations of the foregoing concepts (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figs illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figs. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for predicting a likelihood of dropout and a root dropout cause for a patient under remote monitoring using a dropout prediction system, the method (100) comprising:
obtaining (110), from an electronic patient records database, a plurality of records for a patient under remote monitoring by a care provider;
generating (120) a chronological snapshot of the patient, wherein the chronological snapshot includes a plurality of dropout prediction features extracted from the plurality of records obtained from the electronic patient records database;
generating (130) a likelihood of dropout for the patient by comparing the chronological snapshot of the patient with at least one of: a clinically-validated care pathway, an earlier chronological snapshot of the patient; and one or more similar patient snapshots;
predicting (140) a root dropout cause for the patient based on one or more of the dropout prediction features of the chronological snapshot of the patient;
determining (150) one or more recommended interventions tailored to the patient based on the generated likelihood of dropout and the predicted root dropout cause, wherein the one or more recommended interventions are intended to prevent dropout of the patient from remote monitoring by the care provider; and
presenting (160) to the care provider, via a care provider interface of the dropout prediction system, the one or more recommended interventions.

2. The method (100) of claim 1, wherein generating a likelihood of dropout for the patient includes generating a first likelihood of dropout for the patient corresponding to a first future time and generating a second likelihood of dropout for the patient corresponding to a second future time;
wherein predicting a root dropout cause for the patient includes predicting a first root dropout cause for the patient corresponding to the first future time and predicting a second root dropout cause for the patient corresponding to the second future time; and
wherein the one or more recommended interventions include a first recommended intervention determined based on the first likelihood of dropout generated for the patient and the first root dropout cause predicted for the patient, and a second recommended intervention determined based on the second likelihood of dropout generated for the patient and the second root dropout cause predicted for the patient.

3. The method (100) of claim 2, wherein the first future time is between 1 and 14 days from a current time, and the second future time is between 1 and 6 months from the current time.

4. The method (100) of claim 1, wherein the plurality of records for the patient under remote monitoring by the care provider include at least one of: identification information for the patient; medical history for the patient; treatment history for the patient; medical directives for the patient; unique identifiers/fingerprints of patient's virtual trajectory through the patient interface based on abstraction of virtual movements represented in a virtual trajectory of human-machine interaction within patient interfacing computer program or application; and one or more physiological measurements taken from the patient.

5. The method (100) of claim 4, wherein the plurality of records for the patient under remote monitoring by the care provider further includes at least one of: virtual care solution usage information; a technology affinity measured for the patient; and feedback information from one or more historical or concurrent patients.

6. The method (100) of claim 1, wherein generating a likelihood of dropout for the patient by comparing the chronological snapshot of the patient with one or more similar patient snapshots includes comparing the chronological snapshot of the patient with one or more similar patient snapshots using an unsupervised hierarchical clustering technique.

7. The method (100) of claim 1, wherein the plurality of dropout prediction features extracted from the plurality of records includes at least one of: a response time from the care provider to a patient-initiated action; a history of challenges involving use of a virtual care solution by the patient; clinical trends for the patient; user interaction data for the patient; and portal use data showing frequency of use of specific components over time.

8. The method (100) of claim 1, wherein the root dropout cause for the patient is predicted based on one or more of the dropout prediction features of the chronological snapshot of the patient using an intervention component.

9. The method (100) of claim 8, further comprising:
recording (170), in the electronic patient records database, the implementation of one or more of the recommended interventions by the care provider;
determining (180) an impact of the one or more recommended interventions implemented by the care provider on the plurality of dropout prediction features extracted from the plurality of records; and
updating (190) the intervention component based on the impact determined for the one or more recommended interventions implemented by the care provider.

10. The method (100) of claim 1, further comprising:
determining (141) a level of accuracy for the root dropout cause predicted for the patient; and
if the level of accuracy is below a threshold:
automatically (143) initiating a patient outreach action via a patient interface of the dropout prediction system;
receiving (144), via the patient interface, a response from the patient, wherein the response indicates a true dropout cause for the patient; and
recording (145), in the electronic patient records database, the response; and
updating (146) a dropout prediction engine based on the response received via the patient interface.

11. A dropout prediction system (410) configured to generate a likelihood of dropout and predict a root dropout cause for a patient undergoing remote patient monitoring by a care provider, the system (410) comprising:
an electronic patient records database (402) comprising a plurality of records for a plurality of patients (412, 414, 416) under remote monitoring by the care provider (408);
a dropout prediction engine (563) configured to generate a likelihood of dropout for the patient and to predict a root dropout cause for the patient;
one or more processors (520) configured to: (i) obtain, from the electronic patient records database (402), a plurality of records (404) for the patient under remote monitoring by the care provider (408); (ii) generate a chronological snapshot of the patient, wherein the chronological snapshot includes a plurality of dropout prediction features extracted from the plurality of records (404) obtained from the electronic patient records database (402); (iii) generate the likelihood of dropout for the patient by comparing the chronological snapshot of the patient with at least one of: a clinically-validated care pathway, an earlier chronological snapshot of the patient; and one or more similar patient snapshots; (iv) predict a root dropout cause for the patient based on one or more of the dropout prediction features of the chronological snapshot of the patient; (v) determine one or more recommended interventions tailored to the patient based on the generated likelihood of dropout and the predicted root dropout cause, wherein the one or more recommended interventions are intended to prevent dropout of the patient from remote monitoring by the care provider (408); and (vi) present to the care provider (408), via a care provider interface (418), the one or more recommended interventions; and
a care provider interface (418) configured to present the likelihood of dropout generated for the patient, the root dropout cause predicted for the patient, and the one or more recommended interventions determined for the patient.

12. The dropout prediction system (410) of claim 11, wherein generating a likelihood of dropout for the patient includes generating a first likelihood of dropout for the patient corresponding to a first future time and generating a second likelihood of dropout for the patient corresponding to a second future time;
wherein predicting a root dropout cause for the patient includes predicting a first root dropout cause for the patient corresponding to the first future time and predicting a second root dropout cause for the patient corresponding to the second future time; and
wherein the one or more recommended interventions include a first recommended intervention determined based on the first likelihood of dropout generated for the patient and the first root dropout cause predicted for the patient, and a second recommended intervention determined based on the second likelihood of dropout generated for the patient and the second root dropout cause predicted for the patient.

13. The dropout prediction system (410) of claim 11, wherein the dropout prediction engine (563) is configured to generate a likelihood of dropout for the patient by comparing the chronological snapshot of the patient with one or more similar patient snapshots using an unsupervised hierarchical clustering technique.

14. The dropout prediction system (410) of claim 11, wherein the dropout prediction engine (563) is configured to predict the root dropout cause for the patient based on one or more of the dropout prediction features of the chronological snapshot of the patient using a trained dropout prediction model, the dropout prediction model being trained on a training dataset by a machine learning algorithm.

15. The dropout prediction system (410) of claim 11, one or more processors (520) are further configured to: (vii) record, in the electronic patient records database, the implementation of one or more of the recommended interventions by the care provider (408); (viii) determine an impact of the one or more recommended interventions implemented by the care provider on the plurality of dropout prediction features extracted from the plurality of records; and (ix) update an intervention component (564) based on the impact determined for the one or more recommended interventions implemented by the care provider (408).
